# EUROPEAN PATENT SPECIFICATION

(11) **EP 2 476 420 B1**
(45) Date of publication and mention of the grant of the patent: **26.11.2014**
(21) Application number: 11827039.6
(22) Date of filing: 11.05.2011
(51) Int. Cl.: A61K 31/665, A61K 9/14, A61K 9/51, A61K 47/04, A61P 31/04

(54) **PHARMACEUTICAL COMPOSITION HAVING ANTIMICROBIAL AND FAST-HEALING ACTIVITY FOR EXTERNAL ADMINISTRATION, PROCESS FOR PREPARING SAME**
PHARMAZEUTISCHE ZUSAMMENSETZUNG MIT ANTIMIKROBIELLER UND SCHNELL HEILENDER WIRKUNG ZUR EXTERNEN VERABREICHUNG SOWIE HERSTELLUNGSVERFAHREN DAFÜR
COMPOSITION PHARMACEUTIQUE À ACTION ANTIMICROBIENNE ET CICATRISANTE POUR APPLICATION EXTERNE, ET PROCÉDÉ DE FABRICATION

(30) Priority: 20.09.2010 EA 201001506
(43) Date of publication of application: 18.07.2012
(73) Proprietor: Limonov, Viktor Lvovich, Moscow 121374 (RU)
(72) Inventor: GAIDUL, Konstantin Valentinovich, Novosibirsk 630089 (RU); DUSHKIN, Aleksandr Valerevich, 630117 Novosibirsk (RU); LIMONOV, Viktor Lvovich, Moscow 121374 (RU)
(74) Representative: Morgades y Manonelles, Juan Antonio
(86) International application number: PCT/RU2011/000322
(87) International publication number: WO 2012/039642

(56) References cited:
- EP-A1- 0 556 110
- EP-A2- 0 470 431
- EA-B1- 013 864
- JP-A- 9 183 720
- WANG, YANG: 'Antibiotic-conjugated polyacrylate nanoparticles: New opportunities for development of anti-MRSA agents. Paper 2746' GRADUATE SCHOOL OF THESES AND DISSERTATIONS, [Online] 01 June 2006, page 199 Retrieved from the Internet: <URL:http://scholarcommons.usf.edu/etd/2746 >
- ROSEMARY M.J. ET AL.: 'Investigations of the Antibacterial Properties of Ciprofloxacin@ Si02' LANGMUIR vol. 22, 2006, pages 10125 - 10129

## Description

The present invention relates to antimicrobial pharmaceutical preparations and technologies for the production thereof. It can be used in medicine and veterinary science for prevention and treatment of wound infections of skin and soft tissues, to provide an accelerated wound healing during postoperative period; it can also be used in the pharmaceutical industry for the production of medicaments.

It is known, that the antibiotic with the international nonproprietary name - fosfomycin, which possesses a wide range antimicrobial action on many gram-positive and gram-negative microorganisms, can be successfully used for the treatment of infections of skin, soft tissues, bones and joints by intravenously injecting the parenteral form thereof, which is basically fosfomycin sodium salt [1, 2, 3, 4, 5].

It has been discovered that fosfomycin can penetrate into phagocytes (neurophiles and macrophages), stimulate their phagocytic activity and has a bactericidal action on endocellularly located microorganisms [6,7].

It has also been proved that fosfomycin can decrease the inflammatory response terebrant phase as well as penetrate into biofilms generated by multilayered microbial associations, making them vulnerable and permeable for other antibiotics [8, 9, 10].

Apart from the qualities mentioned above fosfomycin (in its parenteral form) in case of local administration is capable of stimulating processes of hemostasis and angiogenesis, activating chemotaxis of monocytes and fibroblasts in inflammation areas, increasing the quantity of macrophages producing tissue fibronectin; that is why there is a good reason for having patented this antibiotic as a vulnerary medicament for external administration in form of powder, lotion, ointment or any other form [11]. The mentioned patent is the closest prior art document for the proposed pharmaceutical composition and has been considered as a prototype of the present invention.

One of the disadvantages of the prototype is the absence of adsorptive and osmolar properties, which prevent the necessary evacuation of the wound content and the sorption of hystolysis products as well as the products microbial degradation thereby reducing the level of the therapeutic efficiency.

The mentioned invention solves the issue of creating an antimicrobial and vulnerary pharmaceutical composition for external application on basis of using parenteral form of fosfomycin and finely dispersed nanostructured silica dioxide (BHSiO₂) which possesses an improved therapeutic efficiency in case of treatment of infectious inflammatory diseases.

The problem is solved by suggesting to use an antimicrobial and vulnerary pharmaceutical composition for external application, which contains antibiotic fosfomycin as therapeutic agent; the composition is in a powder form and contains a finely dispersed nanostructured silica dioxide with a weight ratio fosfomycin: finely dispersed nanostructured silica dioxide of (25-75 mass. %) : (75-25 mass. %).

The problem can be also solved by suggesting a process for the production of an antimicrobial and vulnerary pharmaceutical composition for external application, comprising mixing fosfomycin and other components according to which fosfomycin in the powder form is mixed with the powder finely dispersed nanostructured silica dioxide in a weight ratio fosfomycin : finely dispersed nanostructured silica dioxide of (25-75 mass. %) : (75-25 mass. %) and the received mixture is subjected to mechanical treatment of by impact and abrasive actions.

Therapeutic efficiency of the proposed pharmaceutical composition will impove, if it the received mixture is subjected to mechanical treatment by impact and abrasive actions to obtain at least 4% of the finely dispersed nanostructured silica dioxide having a size of ≤ 5 micron

To prepare the mentioned pharmaceutical composition, fosfomycin (parenteral form) was used produced by a Spanish company "Ercros". As BHSiO₂ medicament "Polysorb" was used (pharmacological group: enterosorbing solution; active substance: colloidal silica dioxide), produced by Russian company CJSC "Polysorb", containing round-shaped silica dioxide nanoparticles (size 5-20 nm) combined into aggregates (microparticles having irregular shape) with the size of ≤ 90 micron (registration number N_{º} 001140/01-100908).

The selection of the make-up of the composition was based on convertible sorption of fosfomycin molecules by BHSiO₂ micro particles, together with the decrease of the BHSiO₂ particle size during the mechanical activation of its mixtures with fosfomycin substance by an intensive impact and abrasive mechanical process.

BHSiO₂ has been chosen because being different from other substances due to the lack of toxicity and, having absorbing, osmolar and moisture absorbing properties, it is used in medicine for the treatment of infected wounds and it is also included into the make-up of well known vulnerary compositions, which do not contain antibiotic fosfomycin and are prepared by a different method than the one use for the preparation of the claimed composition [12, 13,14,15].

Besides, BHSiO₂ has been chosen because SiO₂ nanoparticles differ in their pharmacologically advantageous biocompatibility, biodistribution, biodegradation and their low-tocxicity properties (regardless of the level of structure porosity). Therefore, they can serve as antibiotic carriers for endocellular delivery of antibiotics into macrophages, which are concentrated in the inflammation areas, i.e. can considerably increase the concentration of antibiotics in the infected tissues, as well as stimulate the antimicrobial activity of those immune system cells what will lead to a significant improvement of the therapeutic efficiency of the antimicrobial preparations in the treatment of skin and soft tissues infectious and inflammatory diseases [16, 17].

The claimed process for the production of the above-mentioned pharmaceutical composition by mechanical activation of the powder mixture of fosfomycin and BHSiO2 with intensive impact and abrasive action allow increasing the percentage of the finely dispersed BHSiO₂ particles (having a size of less than 5 micron) on which fosfomycin molecules are adsorbed and which are mostly phagocyted by macrophages [18].

For this reason the mixture of the above-mentioned materials in weight ratio fosfomycin : BHSiO₂ of (25-75 mass. %) : (75-25 mass. %) is exposed to intensive impact and abrasive mechanical activation process in order to increase the weight percentage of the finely dispersed fraction to up to 40%.

The received powder composition containing finely dispersed BHSiO₂ with convertibly sorbed fosfomycin molecules on their surface, can be used for external application as a powder composition or as a 1-10% water suspension.

To receive the composition mechanochemical method was used consisting in the treatment of mixture of solid components by intensive mechanical impacts - pressure and shearing, mostly carried out in different kind of mills which perform impact and abrasive actions on the substances. The mixture of the solid fosfomycin substance and finely dispersed nanostructured silica dioxide taken in the ratio (25-75 mass.%):(75-25 mass.%) by weight is exposed to mechanical activation in bead mills. The used method for the production of mixtures significantly helps to avoid chemical degradation of the antibiotic and achieve full homogeneity of powder components in comparison with producing the mixture by simple mixing of components or evaporating solutions thereof. As a result a high pharmacological activity of pharmaceutical composition is ensured.

As a quantitative criterion of the minimum necessary mechanical impact it is suitable to use the method of granulometry for the suspension of the obtained compositions. It is necessary that the percentage by weight of the particles having a size of smaller than 5 micron is at least 25%. The mechanical treatment of powder mixtures is performed in rotary, vibrational and planetary mills. As grinding bodies, balls, cores and etc. can be used.

Pharmacological test of the obtained composition with laboratory animals (guiney pigs and rabbits) have showed, that the claimed composition prepared by the claimed method have a higher antimicrobial and vulnerary efficiency comparing to the initial fosfomycin.

Thus, the use of the claimed pharmaceutical composition and the process for the production thereof ensure the following advantages:
1) Clinically significant increase of the effectiveness and quality of the antimicrobial therapy of infections of skin and soft tissues, as well as reduction of the cicatrization period for surgical wounds;
2) Ecological safety, lack of wastes and low price of pharmaceutical production technology.

The claimed invention is illustrated by examples listed below.

### Example N_{º}1. Production of the powder composition: fosfomycin/ BHSiO₂.

The mixture of fosfomycin and BHSiO₂ in weight ratio 3:1, 1:1and 1:3 are being processed in an orbicular rotary mill for 2 and 4 hours. The data of the granulometric make-up of the water suspensions (we used a laser Micro-Sizer 201 granulometer) as well as HPLC analysis of the antibiotics contained in them (in % from the initial substance) are listed in the table N_{º}1.

As you can see from table N_{º}1 the chosen conditions of the production of the claimed composition allow increasing the percentage of the finely dispersed BHSiO₂ fraction (particles size less than 5 micron) up to a certain value (not less than 40% from the total weight) and at the same time avoiding the chemical degradation of the antibiotic.

Fosfomycin sorption rate by BHSiO₂ particles was 20-25%.

**Table N_{º}1**

| **Granulometric make-up of water suspensions, fosfomycin sorption rate and content in different variants of the composition** | | | |
|---|---|---|---|
| Make-up of the composition | Size and content % of BHSiO₂ particles* | | Fosfomycin content (%) |
| | %<2 micron | % < 5 micron | |
| Initial BHSiO₂ | 0,37 | 5,5 | - |
| Fosfomycin:BHSiO₂ (3:1), m/a 2 hours | 8,3 | 40,2 | 98 |
| Fosfomycin:BHSiO₂ (1:1), m/a 2 hours | 12,4 | 45,6 | 99 |
| Fosfomycin: BHSiO₂ (1:3), m/a 4 hours | 14,1 | 44,7 | 97 |

| | | | |
|---|---|---|---|
| *- finely dispersed nanostructured silica dioxide | | | |

### Example N_{º}2. Determination of the therapeutic efficiency of fosfomycin and pharmaceutical compositions.

There has been a research of fosfomycin mechanized for 2 hours and composed of a mixture antibiotic/ BHSiO₂ in weight ratio 1:1, i.e. (50 mass.%) : (50 mass.%).

The experiments were conducted using adult rabbits "Chinchilla" (males, weight 3-3,5 kg) and random bred Guiney pigs (males, weight 0,8-0,9 kg) according to the "Regulations for test animals use" (USSR Ministry of health order supplement #755 from 12.08. 1977).

### Experimental models.

### 1. Incision

There have been made several incisions with a sterile scalpel on the left and right side of the depilated coupling area (1,5% novocain solution was used for local anesthesia); incision 2 cm long and 0,8 cm deep (muscular layer involved) for Guiney pigs and 2cm long and 1 cm deep with (muscular layer involved) for rabbits.

Each day in 8 days period (starting with the very first day) the wounds of the control group of animals were bathed with a sterile physiological solution, were covered with a sterile tissue fixed with a plaster. The wounds of the test group animals were bathed with a sterile physiological solution, then dried and then dusted with fosfomycin sterile powder uniform layer (2-3 mm) or a same layer of the pharmaceutical composition (for guiney pigs), the wounds of the test rabbits were irrigated with a sterile 2,5% fosfomycin solution or sterile 5% pharmaceutical composition suspension, after that they were dried and covered with a sterile tissue fixed with a plaster.

The regenerative process dynamics was monitored during 9 days. The length of the wound open area was measured and a visual evaluation of wound edges and walls, the presence and character of the exudates, necrosis presence was performed. You may see the test results in table N_{º}2 and N_{º}3.

### 2. Infected thermal burn

There have been made a burn of cutaneous covering with a metallic applicator warmed up on the burner flame on the left and right side of the depilated coupling area (1,5% Novocain solution was used for local anesthesia) by attaching it to the skin and holding for 40 seconds. In the center of the ambustial area there has been intracutaneously inserted a daily suspension of 0,8 ml of *Staphylococcus aureus* (ATCC N_{º} 25923 F-49) in an amount of 10¹⁰ CFU/ml.

Each day in 13 days period (starting with the very first day) the burns of the animals control group were bathed with a sterile physiological solution, dried and covered with a sterile tissue fixed with a plaster. The burns of the test animals were irrigated with a sterile 2,5% fosfomycin solution or sterile 5 % suspension of the pharmaceutical composition, after that they were dried and covered with a sterile tissue fixed with a plaster.

The dynamics of burn conditions was monitored during 14 days, the burning wound surface and necrosis area in the center of the burning wound were measured. You may see the results in the table N_{º}4.

The statistical data processing has been performed with a software Statistica 6,0. The experimental data are presented as median (Me), Low and High quartile (LQ-HQ), the significance of the difference has been calculated by means of the Student's t-test at the values of p<0.05.

### Results.

1. In case of incision experiments after 48 hours the following results have been observed in case of Guiney pigs: even wound edges, clear bottom, visible bands and lateral oblique muscles muscular layer, in the wound center a small amount of a sanious exudate was observed, no microbial contamination has been noted. From day 2 to day 9 there has been noted an open wound area reduction. This parameter reflects the regeneration process..
hharmaceutical compositions fosfomycin : BHSiO₂ (in the weight ratio 1:1) significantly increase incisions regeneration process comparing to the control group, taking into consideration the fact that from day 6 the therapeutic efficiency of the composition was significantly higher than fosfomycin.

**Table N_{º} 2**

| **Effect of the fosfomycin and the pharmaceutical composition (powder forms) on incisions regeneration process for Guiney pigs in case of external application.** | | | | |
|---|---|---|---|---|
| Test groups (n - animals q-ty) | | Incision length (cm) Me (LQ-HQ)* | | |
| | | Day 2 | Day 6 | Day 9 |
| 1 | Control | 1,3 | 1,1 | 0,6 |
| | (n=8) | (1,1-1,8) | (0,9 - 1,3) | (0,4-0,7) |
| 2 | Fosfomycin | 0,9 | 0,5 | 0,3 |
| | (n=10) | (0,7-1,3) | (0,3-0,6) | (0,2-0,4) |
| | | | P₁₋₂ <0,02 | P₁₋₂ <0,05 |
| 3 | Fosfomycin : BHSiO₂ (1:1), m/a for 2 hours | 0,6 | 0,3 | 0,15 |
| | | (0,5-0,7) | (0,2-0,4) | (0,1-0,2) |
| | (n=10) | P₁₋₃<0,01 | P₁₋₃ <0,01 | P₁₋₃ <0,01 |
| | | | P₂₋₃ <0,05 | P₂₋₃ <0,02 |

| | | | | |
|---|---|---|---|---|
| *- median, low and high quartiles | | | | |

2. In case of incision experiments after 48 hours the rabbits had results mentioned below: even wound edges, clear bottom, visible bands and lateral oblique muscles muscular layer, in the wound center there was a small amount of sanious exudate, no microbial contamination has been noted. In the following days the wounds (for all test groups) did not differ from each other according to the mentioned parameters. From day 2 to day 9 there has been noted an open wound area reduction. This parameter reflects the regeneration process.
From the data shown in table N_{º}3 you may see that 2,5% fosfomycin solution and 5% pharmaceutical composition water suspension fosfomycin : BHSiO₂ (in the weight ratio 1:1) significantly increase incisions regeneration process compare to the control group, taking into consideration the fact that from day 6 the therapeutic efficiency of the composition was significantly higher than fosfomycin.

**Table N_{º} 3**

| **The effect of fosfomycin and pharmaceutical composition (2,5 % solution and 5 % suspension, respectively) on incisions regeneration process for rabbits in case of external application.** | | | | |
|---|---|---|---|---|
| Test groups (n - animals q-ty) | | Incision length (cm) Me (LQ-HQ)* | | |
| | | Day 2 | Day 6 | Day 9 |
| 1 | Control | 0,6 | 0,5 | 0,35 |
| | (n=8) | (0,5-0.7) | (0,4-0,6) | (0,3-0,4) |
| 2 | Fosfomycin | 0,5 | 0,4 | 0,25 |
| | (n=8) | (0,4-0,6) | (0,3-0,5) | (0,2-0,3) |
| | | | | P₁₋₂<0,05 |
| 3 | Fosfomycin: BHSiO₂ (1:1), m/a for 2 hours | 0,45 | 0,25 | 0,1 |
| | | (0,4-0,5) | (0,2-0,3) | (0,05-0,15) |
| | (n=8) | | P₁₋₃ <0,01 | P₁₋₃<0,01 |
| | | | P₂₋₃<0,05 | P₂₋₃<0,01 |

| | | | | |
|---|---|---|---|---|
| * - median, low and high quartiles | | | | |

3. In case of infected thermal burn wound, 24 hours after the experiment has been started the guiney pigs had a notable induration of the cutaneous covering in the thermal burn area, there has been an edema, there has been noted an opened and dried wheal as well as sanious secretion. The edges of the burning wound are clearly limited from the surrounding unaffected skin. Starting from the experiment day 3 there has been noted a necrosis area in the center of the burning. Starting with experiment day 4 there has been noted a decrease of the burnt area and growth of the necrosis area (with its' further decrease). These parameter reflect the antimicrobial and vulnerary action.

From the data shown in table N_{º}4 you may see that 2,5% fosfomycin solution and 5% water suspension of the pharmaceutical composition fosfomycin : BHSiO₂ (in the weight ratio 1:1) reliably improve the regeneration process of thermal burn infected by *S*. *aureus* comparing to the control group, taking into consideration the fact that from day 6 the therapeutic efficiency of the composition was significantly higher than in case of using fosfomycin.

**Table N_{º} 4**

| **The effect of fosfomycin and pharmaceutical composition (2,5% solution and 5% suspension, respectively) on the regeneration process of thermal burn infected with *S*. *aureus* for guiney pigs in case of external application.** | | | | | |
|---|---|---|---|---|---|
| Test groups (n - animals q-ty) | | Thermal burn area / necrosis area (cm²) | | | |
| | | Day 2 | Day 6 | Day 9 | Day 14 |
| 1 | Control | | | | |
| | (n=8) | 1,2/0,0 | 1,1/0,5 | 1,1/0,5 | 1,0/0,4 |
| 2 | Fosfomycin | | | | |
| | (n=10) | 1,2/0,0 | 0,8/0,3 | 0,7/0,25 | 0,4/0,2 |
| | | | P₁₋₂<0,05/P₁₋₂<0,05 | P₁₋₂<0,05/P₁₋₂<0,02 | P₁₋₂<0,01/P₁₋₂<0,01 |
| 3 | Fosfomycin: BHSiO₂ | 1,2/0,0 | 0,5/0,15 | 0,4/0,1 | 0,15/0,05 |
| | (1:1), m/a for 2 hours | | | | |
| | | | P₁₋₃<0,01 /P₁₋₃ <0,01 | P₁₋₃<0,01/P₁₋₃ <0,01 | P₁₋₃<0,01/P₁₋₃<0,01 |
| | (n=10) | | P₂₋₃<0,05/P₂₋₃<0,01 | P₂₋₃ <0,05 / P₂₋₃ <0,01 | P₂₋₃<0,01/P₂₋₃<0,01 |

Therefore, on the basis of the received test results (using incision and infected *S*. *aureus* thermal burn models), we can come to the conclusion that the suggested pharmaceutical composition of antimicrobial and vulnerary action for external application (fosfomycin/BHSiO₂) has a considerably increased therapeutic effect in case of soft tissues and skin infections treatment comparing to the initial fosfomycin (prototype of the mentioned invention).

### Used literature

1. Frossard M., Joukhadar C., Erovic B.M. et al. Distribution and antimicrobial activity of fosfomycin in the interstitial fluid of human soft tissues // Antimicrob. Agents Chemother. - 2000. - Vol.44. - P. 2728-2732.
2. Legat F.J., Maier A., Dittrich P. et al. Penetration of fosfomycin into inflammatory lesions in patients with cellulitis or diabetic foot syndrome // Antimicrob. Agents Chemother. - 2003. - Vol.47. - P.371-374.
3. Sauermann R., Karch R., Langenberger H. et al. Antibiotic abscess penetration: fosfomycin levels measured in pus and simulated concentration-time profiles // Antimicrob. Agents Chemother. - 2005. - Vol.49. - P. 4448-4454.
4. Schintler M.V., Traunmuller F., Metzler J. et al. High fosfomycin concentrations in bone and peripheral soft tissue in diabetic patients presenting with bacterial foot infection // J. Antimicrob. Chemother. - 2009. - Vol.64. - P.574-578.
5. Fernandez-Valencia J.E., Saban T., Canedo T., Olay T. Fosfomycin in osteomyelitis // Chemotherapy. -1976. - Vol.22. - P.121-134.
6. Traub W.H. Interactions of antimicrobial drugs and combined phagocytic/serum bactericidal activity of defibrinated human blood against Serratia marcescens.III. Beta-lactam antibiotics and fosfomycin // Chemotherapy. -1983. - Vol.29. - P.48-57.
7. Perez-Fernandez P., Herrera I., Martinez P. et al. Enhancement of the susceptibility of Staphylococcus aureus to phagocytosis after treatment with fosfomycin compared with other antimicrobial agents // Chemotherapy. - 1995. - Vol.41. - P.45-49.
8. Matsumoto T., Tateda K., Miyazaki S. et al. Fosfomycin alters lipopolysaccharide-induced inflammatory cytokine production in mice // Antimicrob. Agents Chemother. - 1999. - Vol.43. - P. 697-698.
9. Cai Y., Fan Y., Wang R. et al. Synergistic effects of aminoglicosides and fosfomycin on Pseudomonas aeruginosa in vitro and biofilm infections in a rat model // J. Antimicrob. Chemother. - 2009. - Vol.64. - P. 563-566.
10. Zeitlinger M., Marsik C., Steiner I. et al. Immunomodulatory effects of fosfomycin in an endotoxin model in human blood // J. Antimicrob. Chemother. - 2006. - Vol.59. - P. 219-223.
11. European Patent EP0470431.
12. Clinical chemistry and silica dioxide clinical use // Ed. by A.A. Chuiko, - Kiev: «Naukova dumka», 2003. - 416 p.
13. Patent N_{º}32088 UA (Ukraine).
14. Patent N_{º}33629 UA (Ukraine).
15. Chuiko A., Pentyuk A., Shtat'ko E., Chuiko N. Medical aspects of application of highly disperse amorphous silica // Surface Chemistry in Biomedical and Environmental Science. Edited by J.P.Blitz and V. Gun'ko.II. Mathematics, Physics and Chemistry. - 2006. - Vol.228. - P.191-204.
16. Seleem M.N., Munusamy P., Ranjan A et al. Silica-antibiotic hybrid nanoparticles for targeting intracellular pathogens // Antimicrob. Agents Chemother. - 2009. - Vol.53. - P.4270-4274.
17. Waters K. M., Masiello L.M., Zangar R.C. et al. Macrophage responses to silica nanoparticles are highly conserved across particle sizes // Toxicological Sciences. - 2009. - Vol.107. - P. 553-569.
18. Hamilton R.F., Thakur S.A., Mayfair J.K., Holian A. MARCO mediates silica uptake and toxicity in alveolar macrophages from C57BL/6 mice // J. Biological Chemistry. - 2006. - Vol.281. - P. 34218-34226.

## Claims

1. Antimicrobial and vulnerary pharmaceutical composition for external application containing antibiotic fosfomycin as a therapeutic agent, **characterized in that** it is prepared in form of powder and contains finely dispersed nanostructured silica dioxide in the weight ratio fosfomycin : finely dispersed nanostructured silica dioxide of (25-75% by weight) : (75-25% by weight).

2. The composition according to claim 1, **characterized in that** the percentage of the finely dispersed nanostructured silica dioxide particles having a size of ≤ than 5 micron is at least 40%.

3. The process for the production of the antimicrobial and vulnerary pharmaceutical composition for external administration comprising mixing fosfomycin with other components, **characterized in that** the fosfomycin in form of a powder is mixed with powder-like finely dispersed nanostructured silica dioxide in the weight ratio fosfomycin : finely dispersed nanostructured silica dioxide of (25-75 % by weight) : (75-25 % by weight), and the obtained mixture is subjected to a mechanical treatment by impact and abrasive actions.

4. The process according to claim 3, **characterized in that** the mixture is subjected to a mechanical treatment by impact and abrasive actions so that the percentage of the finely dispersed nanostructured silica dioxide particles having a size of ≤ 5 micron is at least 40%.

## Patentansprüche

1. Antimikrobielle und wundheilende pharmazeutische Zusammensetzung zur äußerlichen Anwendung mit dem Antibiotikum Fosfomycin als therapeutischem Wirkstoff, **dadurch gekennzeichnet, dass** sie in Pulverform zubereitet wird und fein dispergiertes nanostrukturiertes Siliciumdioxid in einem Gewichtsverhältnis von Fosfomycin zu fein dispergiertem nanostrukturiertem Siliciumdioxid von (25-75 Gew.-%) : (75-25 Gew.-%) enthält.

2. Zusammensetzung nach Anspruch 1, **dadurch gekennzeichnet, dass** der Prozentsatz der fein dispergierten nanostrukturierten Siliciumdioxidpartikel, die eine Größe ≤ 5 Mikron besitzen, mindestens 40% beträgt.

3. Verfahren zur Herstellung der antimikrobiellen und wundheilenden pharmazeutischen Zusammensetzung zur äußerlichen Anwendung, das die Vermischung von Fosfomycin mit anderen Inhaltsstoffen umfasst, **dadurch gekennzeichnet, dass** das Fosfomycin in Pulverform mit pulverartigem fein dispergiertem nanostrukturiertem Siliciumdioxid in einem Gewichtsverhältnis von Fosfomycin zu fein dispergiertem nanostrukturiertem Siliciumdioxid von (25-75 Gew.-%) : (75-25 Gew.-%) vermischt wird und die so gewonnene Mischung einer mechanischen Behandlung mit Schlag- und Reibwirkung unterzogen wird.

4. Verfahren nach Anspruch 3, **dadurch gekennzeichnet, dass** die Mischung einer mechanischen Behandlung mit Schlag- und Reibwirkung unterzogen wird, sodass der Prozentsatz der fein dispergierten nanostrukturierten Siliciumdioxidpartikel, die eine Größe ≤ 5 Mikron besitzen, mindestens 40% beträgt.

## Revendications

1. Composition pharmaceutique antimicrobienne et vulnéraire en application externe contenant de la fosfomycine antibiotique à titre d'agent thérapeutique, **caractérisée en ce qu'**elle est préparée sous forme de poudre et contient du dioxyde de silicium nanostructuré finement dispersé dans le rapport en poids de fosfomycine : dioxyde de silicium nanostructuré finement dispersé de (25-75 % en poids) : (75-25 % en poids).

2. Composition conformément à la revendication 1, **caractérisée en ce que** le pourcentage des particules de dioxyde de silicium nanostructuré finement dispersé ayant une taille ≤ à 5 microns est d'au moins 40 %.

3. Procédé pour la production de la composition pharmaceutique antimicrobienne et vulnéraire en application externe, comprenant un mélange de fosfomycine et d'autres composants, **caractérisé en ce que** la fosfomycine en forme de poudre est mélangée à du dioxyde de silicium nanostructuré finement dispersé pulvérulent dans le rapport en poids de fosfomycine : dioxyde de silicium nanostructuré finement dispersé de (25-75 % en poids) : (75-25 % en poids), et le mélange obtenu est soumis à un traitement mécanique par des actions d'impact et d'abrasion.

4. Procédé conformément à la revendication 3, **caractérisé en ce que** le mélange est soumis à un traitement mécanique par des actions d'impact et d'abrasion de sorte que le pourcentage des particules de dioxyde de silicium nanostructuré finement dispersé ayant une taille ≤ à 5 microns est d'au moins 40%.
